# EUROPEAN PATENT SPECIFICATION

(11) **EP 2 606 243 B1**
(45) Date of publication and mention of the grant of the patent: **20.07.2016**
(21) Application number: 11818459.7
(22) Date of filing: 19.08.2011
(51) Int. Cl.: F16B 2/10, A47G 25/14, D06F 55/02, D06F 55/00

(54) **SELF-LOCKING CLAMP**
SELBSTVERSCHLIESSENDE KLEMME
PINCE AUTOBLOQUANTE

(30) Priority: 20.08.2010 SE 1000848
(43) Date of publication of application: 26.06.2013
(73) Proprietor: TRADE-INVENT AB, 112 59 Stockholm (SE); Jonas Ahnme Industridesign, 188 22 Stockholm (SE)
(72) Inventor: EWERLÖF, Göran, S-131 41 Nacka (SE); AHNME, Jonas, S-129 39 Hägersten (SE)
(86) International application number: PCT/SE2011/000151
(87) International publication number: WO 2012/023890

(56) References cited:
- EP-A1- 0 075 761
- JP-A- 2008 100 026
- US-A- 3 616 497
- US-A- 5 285 556
- US-A- 5 490 651

## Description

### Technical field of the invention

The present invention refers to a self-locking clamp comprising two clamping jaws and two shanks connected to the clamping jaws.

### State of the art

A garment clamp is known from US 4,763,390 that is manufactured in one piece in a plastic material. The garment clamp comprises a knee joint at the top that comprises two snap-in links that are mutually connected together by a hinge joint in the form of a thin part of the material that the clothes peg is manufactured from. The garment clamp comprises two shanks connected to the knee joint. Clamping jaws are pivotably connected to the opposite sides of the shanks and are intended to rest against one another in an active position. The garment clamp according to US 4,763,390 is relatively complicated as concerns its construction and the transfer of the knee joint to a snapped-over, locking position must take place by a separate actuation of the knee joint, that is located at a distance from the shanks.

A clamp is known from JP 2008100026 that comprises two shanks and a knee joint at the one ends of the shanks, whereby the knee joint connects these one ends of the shanks. At their opposite ends the shanks have opposing toothings that are shifted against each other when the clamp is made to assume an active position. A material part located between the ends extends between the shanks and functions as a centre of rotation for the shanks. Even in this case the locking of the clamp is relatively complicated since on the one hand a shifting of the toothed ends against one another must take place at the same time as the knee joint must be made to assume a snap-over position.

A self-locking clamp with the features of the preamble of claim 1 is known from US5490651A1.

### Objects and features of the invention

A primary goal of the present invention is to indicate a self-locking clamp that is manufactured in one piece and has an extraordinarily user-friendly locking mechanism.

A further object of the present invention is that it should be able to be manufactured in an extraordinarily rational manner, e.g., by injection moulding.

Yet another object of the present invention is that the locking mechanism is integrated with shanks on the clamp, whereby a constructive simplification takes place at the same time as the managing of the clamp is simplified.

At least the primary object of the present invention is realized by an arrangement obtained by the characterising features defined in the following independent Claim 1. Preferred embodiments of the invention are defined in the dependent claims.

### Brief description of the drawings

Preferred embodiments of the invention will be described in the following with reference made to the attached drawings, in which:
Fig. 1A-1F show a number of views of a first embodiment of a self-locking clamp in accordance with the present invention;
Fig. 2A-2F show a number of views of a second embodiment of a self-locking clamp in accordance with the present invention; and
Fig. 3A-3F show a number of views of a third embodiment of a self-locking clamp in accordance with the present invention.

### Detailed description of preferred embodiments of the invention

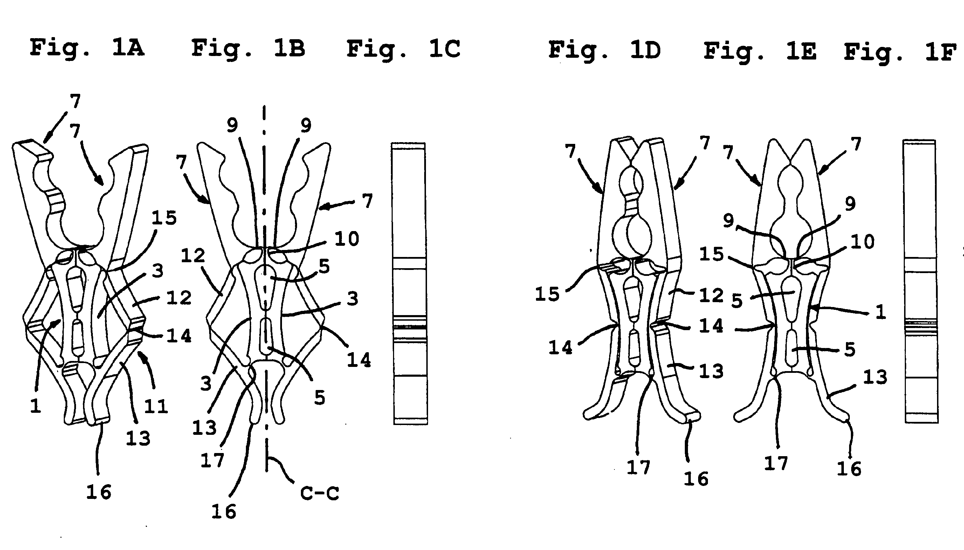

The self-locking clamp in accordance with the invention shown in Fig. 1A-1F is in the form of a clothes peg and comprises a central part 1 distinguished in that it comprises two concave side parts 3. According to the embodiment shown the central part 1 and also the clamp are for the rest symmetrical as regards the centre line C-C extending in dots and dashes in Fig. 1B. According to the embodiment shown the centre part 1 has a longer extension along the centre line C-C than transversely to the centre line C-C. According to the embodiment shown the centre part 1 is not solid but rather provided with through holes 5 in order to achieve a savings of material and also a reduction of weight. It is pointed out in this connection that sufficient material must remain in the centre part 1 so that it will display sufficient rigidity for fulfilling its function. It is also pointed out in this connection that the shaping of the centre part is in no way limited to the shape shown in Fig. 1B.

Two clamping jaws 7 in the self-locking clamp in accordance with the present invention are connected in an articulated manner to the centre part 1, whereby a first flexible connection 9 is effected by a first material part extending between a projecting part 10 in the centre part 1 and the respective clamping jaw 7. The first material parts 9 have relatively little thickness. It should be pointed out in this connection that it should be taken into account during the dimensioning of the first flexible connections/the first material parts 9 that the first connections between the centre part 1 and the clamping jaws 7 should be permanent and at the same time flexible, i.e., that the clamping jaws 7 can pivot in the main plane of the clamp relative to the centre part 1. As concerns the clothes peg shown in Fig. 1A-1F, the parts of the clamping jaws 7 facing one another are formed in a relatively conventional manner, as is already known for clothes pegs. It should be pointed out in this connection that the shaping of the parts facing one another in the clamping jaws 7 is in no way limited to the shaping shown in Fig. 1A-1F.

The self-locking clamp/clothes peg shown in Fig. 1A-1F also comprises two shanks 11, which generally extend along the longitudinal, concave side parts 3 of the central part 1. Each of the shanks 11 comprises two parts 12 respectively 13 that are mutually connected at their adjacent ends by a second flexible connection 14. The end of each of the upper parts 12 which end faces away from the second flexible connection 14 is connected to the associated clamping jaw 7 by a third flexible connection 15, whereby this third connection 15 is connected to a lower, outer part of the clamping jaw 7, i.e., viewed in a plane view the third flexible connection 15 is located at the side of the associated first flexible connection 9.

The lower free end of the lower part 13 in each shank 11 is formed like a gripping part 16, whereby this gripping part 16 in accordance with the embodiment shown is bow-shaped and swung out. In an intermediate part the lower part 13 is connected to a lower corner in centre part 1 by a fourth flexible connection 17.

As concerns the flexible connections 9, 14, 15, 17 in the self-locking clamp in accordance with the present invention these connections can advantageously be formed in a similar manner, i.e., they consist of a relatively thin material part that allows a large number of actuations without wearing out.

The function of the self-locking clamp in accordance with Fig. 1A-1F will now be described. In the position shown in Fig. 1A-1C the self-locking clamp is open, i.e., the clamping jaws 7 are distanced from one another. This is brought about in that the gripping parts 16 in the lower parts 13 of the shanks 11 are moved against each other, e.g., in that the user presses the gripping parts 16 against one another with his one hand. As is most clearly apparent from Fig. 1B, the shanks 11 come to introduce a force on the clamping jaws 7 here that causes the clamping jaws 7 to rotate/pivot around the first flexible connections 9. The parts 12 and 13 of the shanks 11 move into the position shown in Fig. 1b, which is made possible by the second flexible connection 14 between parts 12 and 13. When the self-locking clamp assumes the position shown in Fig. 1A-1C it can be applied, for example, around a clothesline and the article of clothing that is to be carried by the clothesline. When the clothesline and article of clothing are located between the clamping jaws 7 the self-locking clamp is transferred to the position shown in Fig. 1D-1F, which is done by the user by pressing the shanks 11 against one another so that they assume the position shown in Fig. 1D-1E, i.e., the shanks 11 were brought to rest against the concave side parts 3 of the centre part 1, whereby the shanks 11 assumed a so-called snapped-over position.

It can be seen from a study of Fig. 1E that if a separating force is applied on clamping jaws 7, the shanks 11 are pressed harder against the side parts 3 in the centre part 1 on account of the snapped-over position. The clamp according to Fig. 1A-1F is thus self-locking.

In order to open the self-locking clamp the user shifts the gripping parts 16 towards one another and the clamp is transferred to the position shown in Fig. 1A-1C.

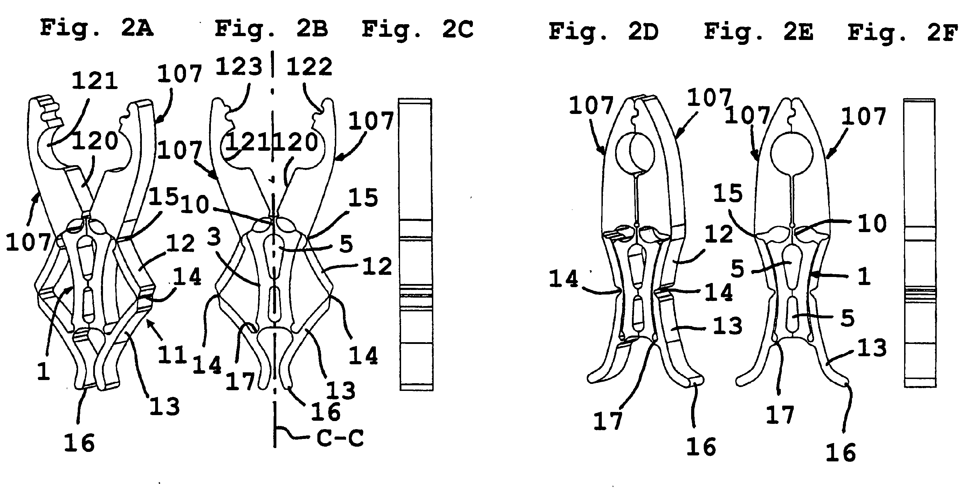

The embodiment of a self-locking clamp in accordance with the present invention shown in Fig. 2A-2F constitutes a forceps. Conceivable areas of application are in medical treatment where a forceps can be used in surgery in order to stop bleeding by clamping blood vessels together. A forceps can also be used in order to clamp tubes together in the case of extracorporeal blood treatment such as dialysis, thoracic surgery, etc.

The clamp according to Fig. 2A-2F is in principle identical to the clamp shown above in accordance with Fig. 1A-1F as concerns the centre part 1, the connections 9, 14, 15, 17 and the shanks 11. For this reason the same reference numerals are used when the parts are the same as the ones used in Fig. 1A-1F. The principle difference that the embodiment according to Fig. 2A-2F has in comparison to the embodiment according to Fig. 1A-1F is the form of clamping jaws 107. As is apparent from Fig. 2A-2F each of the clamping jaws 107 has a plane part 120 located nearest to the central part 1 that is intended to rest against the opposing plane part 120 of the other clamping jaw 107 in the active position. Outside the plane part 120, i.e., further away from central part 1, each of the clamping jaws 107 has a recess 121 that according to the embodiment shown is semicircular in a plane view. In the active position recess 121 forms a circular opening with the corresponding recess 121 in the other clamping jaw 107. In front of recess 121, i.e., further away from central part 1, each of clamping jaws 107 has a toothed part 122, 123 with profilings, whereby the profilings in the one toothed part 122 match the profilings in the other toothed part 123. This is most clearly apparent from Fig. 2D and 2E, where the toothed parts 122, 123 tightly abuts one another.

As regards the self-locking function of the clamp in accordance with Fig. 2A-2F reference is made to what was said previously when the clamp in accordance with Fig. 1A-1F was concerned. As was stated previously, the structural design of the central part 1 and of the shanks 11 is in principle identical for both embodiments.

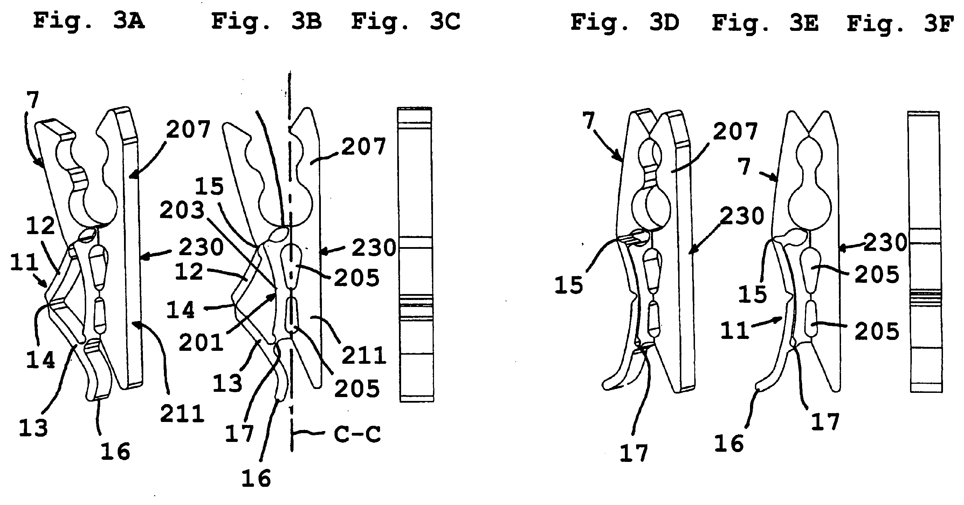

The embodiment of the self-locking clamp according to the present invention and shown in Fig. 3A-3F comprises two halves. Concerning the one half of the clothes peg according to 3A-3F, it is in principle identical to the one half of the clamp/clothes peg shown in 1A-1F. For this reason this one half of the clamp/the clothes peg according to Fig. 3A-3F has been given reference numerals corresponding to the ones in Fig. 1A-1F.

As concerns the central part 201, it is in principle halved in comparison to the central part 1 in accordance with the above-described embodiments, i.e., the central part 201 comprises a concave side part 203.

The clamp/clothes peg according to Fig. 3A-3F also comprises a second half in the form of a rigid/stiff part 230. This rigid/stiff part 230 has a great similarity to the one half of a conventional clothes peg, i.e., the rigid/still part 230 comprises a rigid clamping jaw 207 and a rigid shank 211, whereby the clamping jaw 207 and the shank 211 are integrated with one another. The stiff part 230 is connected to the central part 201, whereby through holes 205 are included in the connection area between the stiff part 230 and the central part 201. It should be pointed out in this connection that the form of the rigid/stiff part 230 is in no way limited to the form shown in Fig. 3A-3F.

As concerns the function of the clamp/clothes peg according to Fig. 3A-3F, the half that is in principle identical to the one half in the embodiment according to Fig. 1A-1F functions in a manner corresponding to this one half in the embodiment according to Fig. 1A-1F. Therefore, reference is made to the above functional description of the embodiment according to Fig. 1A-1F.

As concerns the material in the self-locking clamp in accordance with the present invention, polypropene appears to be a suitable material. The self-locking clamp in accordance with the present invention can be produced, e.g., by injection moulding or extrusion.

### Conceivable modifications of the invention

In the embodiments described above the central part 1; 201 comprises at least one concave side part 3; 203, whereby the flexible shank 11 rests against the concave side part 3; 203 in a snapped-over position. In the framework of the present invention the central part does not have to be provided with concave side parts. By way of an exemplifying and non-limiting measure it can be stated that the side part can be constituted by two plane parts that meet in a tip, i.e., the side part is slightly V-shaped in a plane view.

## Claims

1. A self-locking clamp that comprises two clamping jaws (7; 107; 207) and two shanks (11; 211) connected to the clamping jaws (7; 107; 207), **characterized in that** the clamp comprises a central part (1; 201), that at least the one clamping jaw (7; 107) is fastened in an articulated manner to the central part (1; 201) via a first flexible connection (9), that at least the one shank (11) comprises two parts (12, 13) that are connected in an articulated manner to one another at adjacent ends via a second flexible connection (14), that the shank (11) is fastened in an articulated manner via a third flexible connection (15) to the one clamping jaw (7; 107), that the shank (11) is fastened in an articulated manner via a fourth flexible connection (17) to the central part (1; 201), and that the central part (1; 201) is formed in such a manner that the one shank (11) can assume a snapped-over position adjacent the central part (1; 201).

2. The self-locking clamp according to Claim 1, **characterized in that** the central part (1; 201) comprises at least one concave side part (3), and that this side part (3) faces the one shank (11).

3. The self-locking clamp according to Claim 1 or 2, **characterized in that** the clamp comprises two shanks (11), that each of the shanks (11) comprises two parts that are connected in an articulated manner to one another at adjacent ends via a flexible connection 14.

4. The self-locking clamp according to Claim 3, **characterized in that** the central part (1) comprises concave side parts (3) on both sides.

5. The self-locking clamp according to any of the previous claims, **characterized in that** at least the one shank (11) is provided with a gripping part (16) at its free end.

6. The self-locking clamp according to Claim 1 or 2, **characterized in that** the clamp comprises a rigid part (230) with clamping jaw and shank, and that the rigid part (230) is firmly connected to the central part (201).

7. The self-locking clamp according to any of the previous claims, **c**h**aracterized in that** the clamp is mirror-inverted around a longitudinal centre line (C-C).

8. The self-locking clamp according to any of the previous claims, **characterized in that** the flexible connections (9, 14, 15, 17) are constituted by thin material parts.

9. The self-locking clamp according to Claim 8, **characterized in that** the material in the flexible connections is the same as the material in the rest of the clamp.

## Patentansprüche

1. Selbstverschließende Klemme, die zwei Klemmbacken (7; 107; 207) und zwei mit den Klemmbacken (7; 107; 207) verbundene Schenkel (11; 211) umfasst, **dadurch gekennzeichnet, dass** die Klemme ein Mittelteil (1; 201) umfasst, dass zumindest die eine Klemmbacke (7; 107) über eine erste flexible Verbindung (9) angelenkt an dem Mittelteil (1; 201) befestigt ist, dass zumindest der eine Schenkel (11) zwei Teile (12, 13) umfasst, die an benachbarten Enden über eine zweite flexible Verbindung (14) gelenkig miteinander verbunden sind, dass der Schenkel (11) über eine dritte flexible Verbindung (15) angelenkt an der einen Klemmbacke (7; 107) befestigt ist, dass der Schenkel (11) über eine vierte flexible Verbindung (17) angelenkt an dem Mittelteil (1; 201) befestigt ist, und dass das Mittelteil (1; 201) so geformt ist, dass der eine Schenkel (11) eine eingeschnappte Position angrenzend an das Mittelteil (1; 201) annehmen kann.

2. Selbstverschließende Klemme nach Anspruch 1, **dadurch gekennzeichnet, dass** das Mittelteil (1; 201) mindestens ein konkaves Seitenteil (3) umfasst, und dass dieses Seitenteil (3) dem einen Schenkel (11) zugewandt ist.

3. Selbstverschließende Klemme nach Anspruch 1 oder 2, **dadurch gekennzeichnet, dass** die Klemme zwei Schenkel (11) umfasst, dass jeder der Schenkel (11) zwei Teile umfasst, die an benachbarten Enden über eine flexible Verbindung (14) gelenkig miteinander verbunden sind.

4. Selbstverschließende Klemme nach Anspruch 3, **dadurch gekennzeichnet, dass** das Mittelteil (1) auf beiden Seiten konkave Seitenteile (3) umfasst.

5. Klemme nach einem der vorhergehenden Ansprüche, **dadurch gekennzeichnet, dass** zumindest der eine Schenkel (11) mit einem Griffteil (16) an seinem freien Ende versehen ist.

6. Selbstverschließende Klemme nach Anspruch 1 oder 2, **dadurch gekennzeichnet, dass** die Klemme ein starres Teil (230) mit Klemmbacke und Schenkel umfasst, und dass das starre Teil (230) fest mit dem Mittelteil (201) verbunden ist.

7. Selbstverschließende Klemme nach einem der vorhergehenden Ansprüche, **dadurch gekennzeichnet, dass** die Klemme um eine Mittellängslinie (C-C) herum spiegelverkehrt ist.

8. Selbstverschließende Klemme nach einem der vorhergehenden Ansprüche, **dadurch gekennzeichnet, dass** die flexiblen Verbindungen (9, 14, 15, 17) von dünnen Materialteilen gebildet sind.

9. Selbstverschließende Klemme nach Anspruch 8, **dadurch gekennzeichnet, dass** das Material in den flexiblen Verbindungen dasselbe ist wie das Material in der übrigen Klemme.

## Revendications

1. Pince autobloquante qui comprend deux mâchoires de serrage (7 ; 107 ; 207) et deux branches (11 ; 211) reliées aux mâchoires de serrage (7 ; 107 ; 207), **caractérisée en ce que** la pince comprend une partie centrale (1 ; 201), **en ce qu'**au moins l'une mâchoire de serrage (7 ; 107) est fixée de manière articulée sur la partie centrale (1 ; 201) par le biais d'une première liaison flexible (9), **en ce qu'**au moins l'une branche (11) comprend deux parties (12, 13) qui sont reliées l'une à l'autre de manière articulée à des extrémités adjacentes par le biais d'une deuxième liaison flexible (14), **en ce que** la branche (11) est fixée de manière articulée à l'une mâchoire de serrage (7 ; 107) par le biais d'une troisième liaison flexible (15), **en ce que** la branche (11) est fixée de manière articulée à la partie centrale (1 ; 201) par le biais d'une quatrième liaison flexible (17), et **en ce que** la partie centrale (1 ; 201) est formée de telle manière que l'une branche (11) peut prendre une position enclenchée adjacente à la partie centrale (1 ; 201).

2. Pince autobloquante selon la revendication 1, **caractérisée en ce que** la partie centrale (1 ; 201) comprend au moins une partie latérale (3) concave, et **en ce que** cette partie latérale (3) fait face à l'une branche (11).

3. Pince autobloquante selon la revendication 1 ou 2, **caractérisée en ce que** la pince comprend deux branches (11), **en ce que** chacune des branches (11) comprend deux parties qui sont reliées l'une à l'autre de manière articulée à des extrémités adjacentes par le biais d'une liaison flexible (14).

4. Pince autobloquante selon la revendication 3, **caractérisée en ce que** la partie centrale (1) comprend des parties latérales (3) concaves des deux côtés.

5. Pince autobloquante selon l'une quelconque des revendications précédentes, **caractérisée en ce qu**'au moins l'une branche (11) est dotée d'une partie de préhension (16) à son extrémité libre.

6. Pince autobloquante selon la revendication 1 ou 2, **caractérisée en ce que** la pince comprend une partie rigide (230) avec mâchoire de serrage et branche, et **en ce que** la partie rigide (230) est reliée fixement à la partie centrale (201).

7. Pince autobloquante selon l'une quelconque des revendications précédentes, **caractérisée en ce que** la pince est inversée autour d'une ligne centrale longitudinale (C-C).

8. Pince autobloquante selon l'une quelconque des revendications précédentes, **caractérisée en ce que** les liaisons flexibles (9, 14, 15, 17) sont constituées de parties fines de matériau.

9. Pince autobloquante selon la revendication 8, **caractérisée en ce que** le matériau dans les liaisons flexibles est le même que le matériau dans le reste de la pince.
